# EUROPEAN PATENT APPLICATION

(11) **EP 2 520 379 A2**
(43) Date of publication of application: **07.11.2012**
(21) Application number: 11460064.6
(22) Date of filing: 19.12.2011
(51) Int. Cl.: B09B 3/00, C02F 9/00

(54) **Multi stage waste treatment process**

(30) Priority: 20.12.2010 PL 39337110
(71) Applicant: Komarowski, Leszek, 92-002 Lódz (PL)
(72) Inventor: Komarowski, Leszek, 92-002 Lódz (PL)
(74) Representative: Dziubinska, Joanna

(57) **Abstract**

Waste disposal method, with sorting and homogenization of solid wastes, mixing solid organic wastes with liquids, thermal hydrolysis of wastes, ad anaerobic digestion, whereat wastes delivered to the station are divided into four groups:

- solid wastes (1a),

- liquid wastes (1b),

- sewage sludge (1c) and

- organic waste rich in carbon (1d),

while solid wastes (1a) are subject to crushing and then split into the 3 fractions a) minerals, which are led directly to the municipal landfill (102); b) high-calorific fraction, which is directed to the incinerator (4); and c) biodegradable fraction;

in which the biodegradable fraction c) is mixed with liquid wastes (1b) and/or purified sewage in tanks (8, 9), and the obtained pulp is sent to the thermal hydrolysis in a reactor (10); the sewage sludge (1c) is mixed with purified sewage from a tank (7) and preferably subject to pasteurization, the preferably pasteurized diluted sewage sludge and the substrate exiting the thermal hydrolysis reactor (10) are combined, with addition of excess sludge from a biological water purification stage - and subject to anaerobic digestion in chamber (11), followed by a sedimentation in chamber (13); the in the process of anaerobic digestion obtained biogas is sent to the current generator (103) and a low-calorie excess of biogas is directed to the burner of the incinerator (4), supporting waste incineration; the clear liquid obtained after sedimentation is led to a clear liquid tank (17) and/or tank (7) at the beginning of the process, while the post-sedimentation sludge is treated with coagulants and flocculants, and then filtrated,

a part of the liquid from the clear liquid tank (17) is mixed with organic waste rich in carbon, i.e. the fourth waste group (1d), and is then subject to treatment in a biological treatment stage (18) using active sludge; a part of the active sludge from the biological treatment stage (18) is returned to the biological treatment stage and the excess is sent to the anaerobic digestion chamber (11); the liquid exiting the biological treatment stage (18) is subject to treatment in the chemical treatment station (19).

## Description

The invention relates to the method of thermobiological waste disposal and the installation for the thermobiological waste disposal.

There are known stations and methods of thermobiological waste conversion, where at the beginning of the line there are solid wastes and liquid wastes collecting points (places). Solid wastes on the following positions are subject to crushing and homogenization of size, afterwards wastes are segregated by separating organic from inorganic wastes, such as metals, glass, plastics, textile wastes. Extracted inorganic wastes are sent for further treatment by specialized companies. On the next position the organic solid wastes are mixed with water and liquid wastes such as sewage, whey, manure. The slurry is separated into sand and other rinsed solids, then the sludge is dewatered to 16-17% of dry matter and subject to thermal hydrolysis in known reactor using known method. After the thermal hydrolysis process, the sludge is subject to anaerobic digestion at 40 ° C in the digester compartment, from the top of which is collected biogas arisen during the process. From the bottom of the compartment is collected the digested sludge, which is dehydrated on the next position and is transmitted to the external use in agricultural crops directly to the ground or is being dried and used as biofuel. Water recovered in drainage is recycled to the compartment mixing with solid wastes. Biogas is used to heat water and help to produce steam used in the thermal hydrolysis process, or transmitted to the fuel station to power vehicles.

According to the invention, the method of waste disposal assumes that wastes delivered to the station are preferably divided into four groups: solid wastes, liquid wastes, sewage sludge and waste supporting other wastes treatment. Solid wastes are first subject to crushing, then released of them is mineral fraction which is led directly to the municipal landfill, high-calorific fraction, which is directed to the incinerator and the third fraction, which consists of the remaining biodegradable wastes. The third fraction is mixed with water and / or purified sewage and liquid wastes, and the obtained pulp is sent to the thermal hydrolysis in the reactor. Sewage sludge is mixed with water and / or purified sewage and preferably subject to pasteurization, afterwards they are combined with the substrate after thermal hydrolysis, and preferably with excessive sludge from the biological stage of purification and subject to the anaerobic digestion and the sedimentation. Obtained in the process of anaerobic digestion biogas is sent to the current generator and a low-calorie excess of biogas is directed to the burner of the incinerator supporting waste incineration. The clear liquid obtained after sedimentation is led to the clear liquid tank and / or tank at the beginning of the process, while the post-sedimentation sludge is treated with coagulants and flocculants, and then filtrated, preferably in the filter presses. Post-filtration sludge is stored in the form of a cake and post-filtration effluent is directed to a clear liquid tank. The part of a clear liquid is mixed with supporting wastes, and then subject to treatment in biological stage of treatment, using active sludge, while the part of active sludge from this stage is returned to the biological treatment stage and the excess is sent to anaerobic digestion. The liquid obtained after biological treatment is subject to chemical treatment and then obtained purified wastes in part are returned and used to dilute another portion of wastes at the beginning of the process and the excess is discharged into the river. Sludge from chemical purification shall be drained, preferably centrifuged and the resulting liquid is recirculated back to the chemical treatment, while sludge from draining is added to the filter cake. Cake is dried and sent to be used as a fertilizer or dried and sent to be burned in an incinerator with a high caloric fraction of solid wastes.

The pulp transferred to thermal hydrolysis reactor contains 8-12% of dry solid wastes. Anaerobic digestion is preferably carried out in three steps, while in the first stage thermophilic fermentation is carried out at 55 ° C for 3-5 days, then in the second stage a mesophilic fermentation is carried out at 35-37 ° C during 8-20 days, and the third stage carries a refermentation at 20-30 ° C for up to 14 days, afterwards sedimentation is carried out during 6-12 hours. Biological treatment is carried out in two stages, one stage consist of subjecting a clear liquid with a supporting waste to denitrification and nitrification in the second stage, and then separation is made in the secondary settling tank. Heat to hydrolysis, pasteurization, anaerobic fermentation and drying of the cake is taken from the incinerator of the exhaust and cooling systems of current generating devices.

Installation for the waste disposal according to the invention preferably consists of four chambers, the first of which is for solid wastes, the second for liquid wastes, the third is for sewage sludge and fourth for wastes supporting treatment of other wastes. The chamber of solid wastes through the shredder and the separator is connected to the incinerator, and further, through the mixing point where, after mixing with water and purified wastes the pulp is produced, with a thermal hydrolysis reactor. The chamber for liquid wastes and tank of water and purified sewage are also connected with another mixing point to allow the manufacture of pulp that is transferred to the reactor. The chamber of sewage sludge, through another mixing point, which is also supplied from the tank with water and purified sewage, is connected to the entrance of the chamber of anaerobic fermentation and the entrance is also connected to the exit of the thermal hydrolysis reactor. At the exit of the anaerobic digestion chamber there is a sedimentation chamber, which at the exit has a pipeline for clear liquid, by which clear liquid is transported into the clear liquid tank and / or tank at the beginning of the process. Sedimentation chamber has also the exit to transport the sludge to the station of polyelectrolytes. Sludge from polyelectrolytes station is received by the filter machine preferably by a filter press, the post-filtration effluent is transported to the clear liquid tank and the sludge is transported to a landfill as a fertilizer cake. Supporting waste chamber is connected to the biological treatment stage to the entrance of which clear liquid is also transported and the active sludge recycled from the exit of the biological treatment stage. Excessive sludge is also transported to the anaerobic fermentation chamber entrance, which is connected to the exit of biological treatment stage. The liquid from the biological treatment stage is transported by pipeline to chemical treatment position, from which purified sewage are collected by pipeline to the water and treated sewage tank and the excess of treated sewage is discharged by pipeline into the river. The sludge after the chemical treatment is subject to draining, preferably in a centrifuge, from which the obtained liquid is returned to the entrance of the chemical treatment position. The sludge is discharged to landfill of a cake. Anaerobic digestion chamber has an installation for receiving a biogas that is transported to the current generator. Low-calorie part of biogas is distributed by pipeline to the burner of incinerators of high-calorie wastes. From incinerators and from the exhaust and cooling systems of current generators, the thermal installation brings the heat to the thermal hydrolysis reactor and to sterilization of sewage sludge, as well as to the fermentation chamber and the dryer of a fertilizer cake.
Anaerobic digestion chamber is preferably made of in the first stage of thermophilic fermentation chamber, connected in series with located on the second stage the mesophilic fermentation chamber and located at the other end of series the refermentation tank. The chamber of the sewage sludge is connected to the chamber for anaerobic fermentation through the sterilizer. Biological purification stage consists of a denitrifying chamber, nitrifying chamber and in the exit of the secondary settling tank. The burner of the incinerator is connected with a pipe with low-calorie biogas received from anaerobic digestion process.
Disposal of waste according to the invention, using a system of this invention, it is almost completely waste-free and very economical. Savings are mainly achieved by the use of one group of wastes as active ingredients to eliminate wastes, as well as by own heat production, both for use in the process as well as for sale to the heating network. Small is the consumption of water in the process of waste disposal, practically water is used in a closed circuit and excess is discharged after cleaning to the river. One of the final product is also ideal fertilizer for agriculture, odorless, free of all pathogens without the possibility of reactivation or growth of bacteria. Obtained from the process biogas is used to drive the motor of current generator, which means that the current passed to the national grid is produced from renewable sources. Some current is used by the installation for waste disposal. Low-calorie biogas, which in known systems is burned in the flare, in the present invention is used to support burning of wastes in the incinerator.

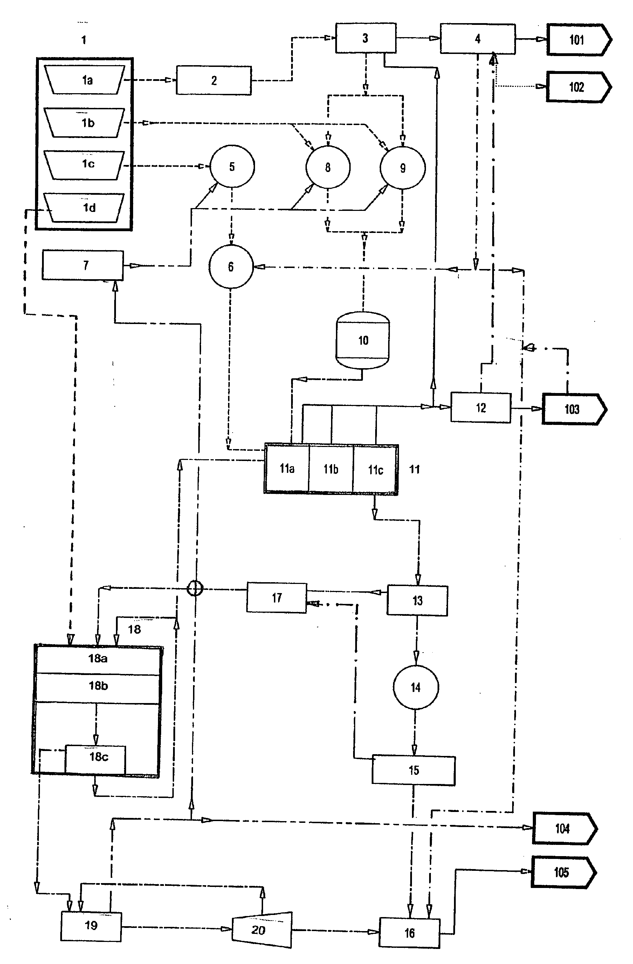

Installation for the disposal of waste of the present invention is shown in the drawing, which
schematically shows the various elements of the installation. As shown, the wastes collection point 1 is formed by four chambers 1a, 1b, 1c and 1d, in one 1a solid wastes are accepted, the second 1b is designed for liquid wastes, the third 1c for sewage sludge and fourth 1d for wastes supporting other wastes treatment. Solid wastes 1a are solid municipal easy- and hard-degradable wastes, separated at municipal landfills, out-dated food wastes, post-mortem solid wastes, green wastes, agricultural products. 1b liquid wastes are whey, manure, post-mortem liquid wastes of a content of dry matter greater than 8%. Sewage sludge 1c is dehydrated waste collected from sewage treatment of a content of dry matter of 12-30%. Supporting wastes 1d are organic solid and / or liquid wastes, easy-degradable, such as wastes rich in simple alcohols, starch and other sugars, rich in nitrogen. As mentioned above, supporting wastes 1d are used to assist in the process of the purification of other wastes, as it will be explained more closely hereafter. Solid wastes chamber 1a is through the shredder 2 and the separator 3 combined with incinerator 4. In incinerators 4 are burned separated from wastes high-caloric fractions, such as foil, paper, bone, wood. Separated mineral ingredients such as sand, scrap metal, glass and ash from the incinerator 4, are transmitted directly to a landfill 102, where they are collected by specialized companies for further use. The third separated fraction is biodegradable wastes, which are transmitted to the mixing point where the pulp is produced. For the production of the pulp used is water mixed with purified sewage, transported to the mixing point from the tank 7, in which water is collected with purified sewage during the process. The pulp is accumulated in the intermediate tanks 8 and 9, from where it is sent to the thermal hydrolysis reactor 10. Substrate from the reactor 10, and sewage sludge 1c from the third chamber are transported to the anaerobic fermentation chamber 11, while the pulp created from sewage sludge 1c and water mixed with purified sewage from the tank 7 is accumulated in the intermediate tank 5, and afterwards the pulp is subject to sterilization in the sterilizer 6. Anaerobic digestion chamber 11 consists of three parts 11a, 11b, 11c, in the first 11a thermophilic fermentation is carried out at 55 ° C for 3-5 days and then in the second stage 11b mesophilic fermentation is carried out at 35-37 ° C during 8-20 days, and in the third stage 11c referementation is conducted at 20-30 ° C for up to 14 days. After the anaerobic digestion in the tank 13 the sedimentation of a slurry is carried out in 10-12 hours. A clear liquid obtained during the sedimentation is sent to the clear liquid tank 17 and / or tank 7 at the beginning of the process, and sludge from the sedimentation tank 13 is subject to coagulants and flocculants action in polyelectrolyte station 14. Small parts of sedimentation sludge under the action of coagulants and flocculants link into larger parts, which easier to filter in presses 15, to which the wastes are transferred from the polyelectrolytes station 14. Postfiltration sludge is removed from the presses 15 directly to the fertilizer cake landfill 16, where it is dried and then transferred to a landfill 105 from where it is collected by the farmers or to burning in incinerators 4. Postfiltration effluent is passed to the clear liquid tank 17. Clear liquid is mixed with supporting wastes 1d, and afterwards is subject to biological treatment in biological treatment stage, the clear liquid from the tank 17 is directed to the tank 7 at the beginning of the process. During biological purification is also used the part of the active sludge, which is recycled from this stage to the re-biological treatment, the remaining part of the active sludge (excess) is sent to anaerobic digestion. Biological purification stage 18 consists of denitrifying chamber 18a and nitrifying chamber 18b and the secondary settling tank 18c. The liquid obtained after biological purification is received from the secondary settling tank 18c and is subject to chemical purification in chemical treatment station 19. Purified sewage obtained there, in part are recycled to the tank 7 and used to dilute another portion of wastes at the beginning of the process and the excess is discharged into the river 104. Sludge from the chemical purification 19 is subject to draining in a centrifuge 20 and the obtained liquid is recirculated again to the chemical treatment 19, while drained sludge is added to the filter cake (fertilizer) 16. To support incineration of wastes in the incinerator 4 from biogas tank 12 is collected a low caloric excess which is administered directly to the burner of the incinerator 4, where it is burned instead of in flares. The process uses the heat generated from incineration 4 and received from the exhaust and cooling systems of current generating devices 103. This heat is transferred to the hydrolysis reactor 10, pasteurization in sterilizer 6, anaerobic digestion in the chamber 11 and drying the postfiltration cake (fertilizer) 16 and the excess is sold to a heat network 101.

## Claims

1. Waste disposal method, with sorting and homogenization of solid wastes, mixing solid organic wastes with liquids, thermal hydrolysis of wastes, anaerobic digestion, **characterized in that** wastes delivered to the station are preferably divided into four groups: solid wastes (1a), liquid wastes (1b), sewage sludge (1c) and waste supporting other waste's treatment (1d), while solid wastes (1a) are first subject to crushing, then released of them is mineral fraction which is led directly to the municipal landfill, high-calorific fraction, which is directed to the incinerator (4) and the third fraction, which consists of the remaining biodegradable wastes, is mixed with water and / or purified sewage and liquid wastes (1c), and the obtained pulp is sent to the thermal hydrolysis in the reactor (10), while the sewage sludge is mixed with water and / or purified waste and preferably subject to pasteurization, afterwards they are combined with the substrate after thermal hydrolysis, and preferably with excessive sludge from biological stage of purification and subject to anaerobic digestion and sedimentation and obtained in the process of anaerobic digestion biogas is sent to the current generator (103) and a low-calorie excess of biogas is directed to the burner of the incinerator (4) supporting waste incineration and the clear liquid obtained after sedimentation is led to the clear liquid tank (17) and / or tank (7) at the beginning of the process, while the post-sedimentation sludge is treated with coagulants and flocculants, and then filtrated, preferably in the filter presses (15) and post-filtration sludge is stored in the form of a cake (16) and post-filtration effluent is directed to a clear liquid tank (17), while the part of a clear liquid is mixed with supporting wastes (1d), and then subject to treatment in biological stage of treatment, using active sludge, while the part of active sludge from this stage is returned to the biological treatment stage and the excess is sent to anaerobic digestion. The liquid obtained after biological treatment is subject to chemical treatment and then obtained purified wastes in part are returned to the tank (7) and used to dilute another portion of wastes at the beginning of the process and the excess is discharged into the river (104), whereas the sludge from chemical purification shall be drained, preferably in centrifuge (20) and the obtained liquid is recirculated back to the chemical treatment, while sludge from draining is added to the filter cake (16), while the cake (16) is dried and sent to be used as a fertilizer or dried and sent to be burned in an incinerator (4) with a high caloric fraction of solid wastes.

2. Waste disposal method according to claim 1, **characterized in that** the pulp transferred to thermal hydrolysis reactor (10) contains 8-12% of dry solid wastes.

3. Waste disposal method according to claim 1, **characterized in that** anaerobic digestion is preferably carried out in three steps, while in the first stage (11a) thermophilic fermentation is carried out at 55 ° C for 3-5 days, then in the second stage (11b) a mesophilic fermentation is carried out at 35-37 ° C during 8-20 days, and the third stage (11c) carries a refermentation at 20-30 ° C for up to 14 days, afterwards sedimentation is carried out during 6-12 hours

4. Waste disposal method according to claim 1, **characterized in that** the biological treatment is carried out in two stages, one stage (18a) consist of subjecting a clear liquid with a supporting waste to denitrification and nitrification in the second stage (18b), and then separation is made in the secondary settling tank (18c).

5. Waste disposal method according to claim 1, **characterized in that** the heat to hydrolysis, pasteurization, anaerobic fermentation and drying of the cake is taken from the incineration (4) of the exhaust and cooling systems of current generating devices (103)

6. Installation for waste disposal, with solid wastes and liquid wastes collection points (places), the sorting chamber, shredder for homogenization of solid wastes, point mixing solid and liquid wastes, thermal hydrolysis chamber and fermentation chamber, **characterized in that** solid and liquid wastes collection point (1) preferably consists of four chambers (1a, 1b, 1c, 1d), the first of which is for solid wastes (1a), the second for liquid wastes (1b), the third is for sewage sludge (1c) and fourth for wastes supporting treatment of other wastes (1d), whereas the chamber of solid wastes (1a) through the shredder (2) and the separator (3) is connected to the incinerator (4), and further, through the mixing point where, after mixing with water and purified wastes from the tank (7), the pulp is produced, which is collected in intermediary tanks (8) and (9), with a thermal hydrolysis reactor (10), while the chamber for liquid wastes (1b) and tank (7) of water and purified sewage are also connected with another mixing point to allow the manufacture of the pulp, while the chamber of sewage sludge (1c), through another mixing point, which is also supplied from the tank (7) with water and purified sewage, by the intermediary tank (5) is connected to the entrance of the chamber of anaerobic fermentation (11) and the entrance is also connected to the exit of the thermal hydrolysis reactor (10), while at the exit of the anaerobic digestion chamber (11) there is a sedimentation chamber (13), which at the exit has a pipeline for clear liquid, by which clear liquid is transported into the clear liquid tank (17) and / or tank at the beginning of the process and sedimentation chamber (13) has also the exit to transport the sludge to the station of polyelectrolytes (14), to which coagulants and flocculants are distributed from the tank, while the sludge from polyelectrolytes station (14) is received by the filter machine preferably by a filter press (15), the post-filtration effluent is transported to the clear liquid tank (17) and the sludge is transported to a landfill as a fertilizer cake (16), while the supporting waste chamber (1d) is connected to the biological treatment stage (18) to the entrance of which clear liquid is also transported and the active sludge recycled from the exit of the biological treatment stage (18), while the excessive active sludge is also transported to the anaerobic fermentation chamber entrance (11), which is connected to the exit of biological treatment stage and the liquid from the biological treatment stage (18) is transported by pipeline to chemical treatment position (19), from which purified sewage are collected by a pipeline to the water and treated sewage tank (7) and the excess of treated sewage is discharged by a pipeline into the river (104), whereas the sludge after the chemical treatment is subject to draining, preferably in a centrifuge (20), from which the obtained liquid is returned to the entrance of the chemical treatment position (19) and the sludge is discharged to landfill of a cake (16), while the anaerobic digestion chamber (11) has an installation for receiving a biogas that is transported to the tank (12), while low-calorie part of biogas is distributed by a pipeline to the burner of the incinerator (4) of high-caloric wastes and from the incinerator and from the exhaust and cooling systems of current generators (103), the thermal installation brings the heat to the thermal hydrolysis reactor (10) and to sterilizer of sewage sludge (6), as well as to the fermentation chamber (11) and the dryer of a fertilizer cake (16).

7. Installation for waste disposal according to claim 6, **characterized in that** the anaerobic digestion chamber (11) is preferably made of the thermophilic fermentation chamber (11a) in the first stage, connected in series with located on the second stage the mesophilic fermentation chamber (11b) and located at the other end of series the refermentation tank (11c).

8. Installation for waste disposal according to claim 6, **characterized in that** chamber of sewage sludge (1c) is connected to the anaerobic digestion chamber (11) through the sterilizer (6).

9. Installation for waste disposal according to claim 6, **characterized in that** the biological treatment stage (18) consists of a denitrifying chamber (18a), nitrifying chamber (18b) and in the exit of the secondary settling tank (18c).

10. Installation for waste disposal according to claim 6, **characterized in that** to the burner of the incinerator (4) from the tank (12) is distributed by a pipeline a low-calorie biogas received from anaerobic digestion process.
